(19) Europäisches Patentamt
European Patent Office
Office européen des brevets

(11) Veröffentlichungsnummer: **0 538 692 A1**

(12) # EUROPÄISCHE PATENTANMELDUNG

(21) Anmeldenummer: **92117263.1**

(51) Int. Cl.[5]: **C07D 207/16**, A61K 31/40

(22) Anmeldetag: **09.10.92**

(30) Priorität: **22.10.91 DE 4134756**

(43) Veröffentlichungstag der Anmeldung: **28.04.93 Patentblatt 93/17**

(84) Benannte Vertragsstaaten: **AT BE CH DE DK ES FR GB GR IE IT LI LU NL PT SE**

(71) Anmelder: **BAYER AG**
**W-5090 Leverkusen 1 Bayerwerk(DE)**

(72) Erfinder: **Kunisch, Franz, Dr.**
**Zum Hahnenberg 20**
**W-5068 Odenthal-Glöbusch(DE)**
Erfinder: **Mittendorf, Joachim, Dr.**
**Pahlkestrasse 3**
**W-5600 Wuppertal 1(DE)**
Erfinder: **Plempel, Manfred, Dr.**
**Zwengenbergerstrasse 3c**
**W-5657 Haan 1(DE)**
Erfinder: **Militzer, Hans-Christian, Dr.**
**Leuchter Gemark 2**
**W-5060 Bergisch Gladbach 2(DE)**

(54) **Verwendung von teilweise bekannten substituierten Pyrrolidinen als Arzneimittel, neue Wirkstoffe und Verfahren zu ihrer Herstellung.**

(57) Die vorliegende Erfindung betrifft substituierte Pyrrolidine der allgemeinen Formel (I)

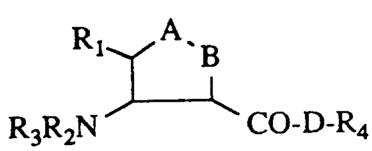

(I)

in welcher A, B, $R_1$, $R_2$, $R_3$, D und $R_4$ die in der Beschreibung angegebene Bedeutung haben, die Verwendung von teilweise bekannten substituierten Pyrrolidinen als Arzneimittel, insbesondere als antimykotische Mittel, neue Wirkstoffe und Verfahren zu ihrer Herstellung.

EP 0 538 692 A1

Die Erfindung betrifft die Verwendung von teilweise bekannten substituierten Pyrrolidinen als Arzneimittel, insbesondere als antimykotische Mittel, neue Wirkstoffe und Verfahren zu ihrer Herstellung.

Pyrrolidincarbonsäurederivate sind aus vielen Publikationen bekannt [vgl. z.B. J. Heterocycl. Chem. 12 (3), 595-6; Liebigs Ann. Chem. (6) 1073-88; Tetrahedron 27 (1971), 4681-6].

Es wurde nun gefunden, daß die substituierten Pyrrolidine der allgemeinen Formel (I)

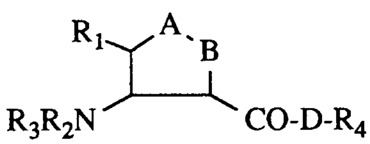

(I),

in welcher

A und B stets verschieden sind und für eine Gruppe der Formel $-CHR^5$ oder $-NR^6$ stehen,
worin

$R^5$ Wasserstoff oder geradkettiges oder verzweigtes Alkyl mit bis zu 8 Kohlenstoffatomen bedeutet, das gegebenenfalls durch Halogen, Hydroxy, Phenyl, Carboxy oder durch geradkettiges oder verzweigtes Alkoxy oder Alkoxycarbonyl mit jeweils bis zu 6 Kohlenstoffatomen substituiert ist,

$R^6$ die unten angegebene Bedeutung von $R^2$ hat und mit dieser gleich oder verschieden ist, oder Methoxycarbonyl bedeutet,

$R^1$ für Wasserstoff oder für geradkettiges oder verzweigtes Alkyl mit bis zu 8 Kohlenstoffatomen steht, das gegebenenfalls 1- bis 2-fach gleich oder verschieden durch Halogen, Hydroxy, Phenyl, Carboxy oder durch geradkettiges oder verzweigtes Alkoxy, Acyl oder Alkoxycarbonyl mit jeweils bis zu 6 Kohlenstoffatomen oder durch eine Gruppe der Formel $-NR^7R^8$ substituiert ist,
worin

$R^7$ und $R^8$ gleich oder verschieden sind und Wasserstoff, Phenyl oder geradkettiges oder verzweigtes Alkyl mit bis zu 6 Kohlenstoffatomen bedeuten,

$R^2$ für Wasserstoff oder
für geradkettiges oder verzweigtes Alkyl mit bis zu 8 Kohlenstoffatomen steht, das gegebenenfalls 1- bis 2-fach gleich oder verschieden durch Hydroxy, Formyl oder durch geradkettiges oder verzweigtes Acyl mit bis zu 6 Kohlenstoffatomen oder durch Phenyl oder Benzoyl substituiert ist, die gegebenenfalls bis zu 2-fach gleich oder verschieden durch Halogen, Nitro, Cyano, oder durch geradkettiges oder verzweigtes Alkyl oder Alkoxy mit jeweils bis zu 6 Kohlenstoffatomen substituiert sind,
oder
für geradkettiges oder verzweigtes Acyl mit bis zu 8 Kohlenstoffatomen, oder
für tert. Butyloxycarbonyl (Boc) oder Benzoyl steht, das gegebenenfalls wie oben beschrieben substituiert ist, oder
für eine Gruppe der Formel $-SO_2R^9$ steht,
worin

$R^9$ geradkettiges oder verzweigtes Alkyl mit bis zu 8 Kohlenstoffatomen, Benzyl oder Phenyl bedeutet, wobei letztere gegebenenfalls bis zu 3-fach gleich oder verschieden durch Halogen, Hydroxy, Nitro, Cyano, Trifluormethyl, Trifluormethoxy oder durch geradkettiges oder verzweigtes Alkyl, Alkoxy oder Alkoxycarbonyl mit jeweils bis zu 6 Kohlenstoffatomen, Carboxy oder durch die oben aufgeführte Gruppe $-NR^7R^8$ substituiert sind,
worin

$R^7$ und $R^8$ die oben angegebene Bedeutung haben,
für Phenyl steht, das gegebenenfalls bis zu 3-fach gleich oder verschieden durch Halogen, Hydroxy, Nitro, Trifluormethyl, Trifluonmethoxy, geradkettiges oder verzweigtes Alkyl, Acyl, Alkoxy oder Alkoxycarbonyl mit jeweils bis zu 6 Kohlenstoffatomen oder durch eine Gruppe der Formel $-NR^7R^8$ oder $-SO_2R^9$ substituiert ist,
worin

$R^7$, $R^8$ und $R^9$ die oben angegebene Bedeutung haben,

$R^3$ für Wasserstoff oder für geradkettiges oder verzweigtes Alkyl mit bis zu 8 Kohlenstoffatomen steht, das gegebenenfalls durch Phenyl substituiert ist, oder

$R^2$ und $R^3$ gemeinsam für den Rest der Formel $=CHR^{10}$ stehen,
worin
$R^{10}$ die oben angegebene Bedeutung von $R^5$ hat und mit dieser gleich oder verschieden ist,
D für ein Sauerstoff- oder Schwefelatom oder für die >NH-Gruppe steht,
$R^4$ für Wasserstoff oder für geradkettiges oder verzweigtes Alkyl mit bis zu 8 Kohlenstoffatomen oder Phenyl steht, wobei letztere gegebenenfalls bis zu 3-fach gleich oder verschieden durch Hydroxy, Halogen, Nitro, Cyano, Carboxy, Trifluormethyl, Trifluormethoxy, durch geradkettiges oder verzweigtes Alkoxy, im Fall von Phenyl auch durch Alkyl, Acyl oder Alkoxycarbonyl mit jeweils bis zu 6 Kohlenstoffatomen oder durch eine Gruppe der Formel $-NR^7R^8$ oder $-SO_2R^9$ substituiert sind,
worin
$R^7$, $R^8$ und $R^9$ die oben angegebene Bedeutung haben,
oder für den Fall, daß D für die >NH-Gruppe steht,
$R^4$ für die Gruppe der Formel $-SO_2R^9$ steht,
worin
$R^9$ die oben angegebene Bedeutung hat,

überraschenderweise eine starke antimikrobielle, insbesondere starke antimykotische Wirkung gegen Dermatophyten, Sproßpilze und biphasische Pilze zeigen und somit geeignet sind zur Verwendung bei der Bekämpfung der Dermatomykosen und Systemmykosen.

Auch die physiologisch unbedenklichen Säureadditions-Salze und Metallsalzkomplexe der Verbindungen der allgemeinen Formel (I) sowie die Racemformen, die Antipoden, die Diastereomerengemische und die einzelnen Isomere sind bevorzugt für diese Verwendung.

Bevorzugt verwendet werden Verbindungen der allgemeinen Formel (I), in welcher

A und B stets verschieden sind und
für eine Gruppe der Formel $-CHR^5$ oder $-NR^6$ stehen,
worin
$R^5$ Wasserstoff oder geradkettiges oder verzweigtes Alkyl mit bis zu 6 Kohlenstoffatomen bedeutet, das gegebenenfalls durch Halogen, Hydroxy, oder durch geradkettiges oder verzweigtes Alkoxy oder Alkoxycarbonyl mit jeweils bis zu 4 Kohlenstoffatomen substituiert ist,
$R^6$ die unten angegebene Bedeutung von $R^2$ hat und mit dieser gleich oder verschieden ist, oder Methoxycarbonyl bedeutet,
$R^1$ für Wasserstoff oder für geradkettiges oder verzweigtes Alkyl mit bis zu 6 Kohlenstoffatomen steht, das gegebenenfalls durch Halogen, Hydroxy, durch geradkettiges oder verzweigtes Alkoxy, Acyl oder Alkoxycarbonyl mit jeweils bis zu 4 Kohlenstoffatomen oder durch eine Gruppe der Formel $-NR^7R^8$ substituiert ist,
worin
$R^7$ und $R^8$ gleich oder verschieden sind und Wasserstoff oder geradkettiges oder verzweigtes Alkyl mit bis zu 4 Kohlenstoffatomen bedeuten,
$R^2$ für Wasserstoff oder
für geradkettiges oder verzweigtes Alkyl mit bis zu 6 Kohlenstoffatomen steht, das gegebenenfalls 1- bis 2-fach, gleich oder verschieden durch Hydroxy, Formyl oder durch geradkettiges oder verzweigtes Acyl mit bis zu 4 Kohlenstoffatomen oder durch Phenyl oder Benzoyl substituiert ist, die gegebenenfalls durch Halogen, Nitro, Cyano, oder durch geradkettiges oder verzweigtes Alkyl oder Alkoxy mit jeweils bis zu 4 Kohlenstoffatomen substituiert sind,
oder
für geradkettiges oder verzweigtes Acyl mit bis zu 6 Kohlenstoffatomen oder für tert. Butyloxycarbonyl (Boc) oder Benzoyl steht, das gegebenenfalls wie oben beschrieben substituiert ist, oder
für eine Gruppe der Formel $-SO_2R^9$ steht,
worin
$R^9$ geradkettiges oder verzweigtes Alkyl mit bis zu 6 Kohlenstoffatomen, Phenyl oder Benzyl bedeutet, wobei letztere gegebenenfalls bis zu 2-fach gleich oder verschieden

durch Halogen, Hydroxy, Nitro, Cyano, Trifluormethyl, Trifluormethoxy oder durch geradkettiges oder verzweigtes Alkyl oder Alkoxy mit jeweils bis zu 4 Kohlenstoffatomen oder durch die oben aufgeführte Gruppe der Formel $-NR^7R^8$ substituiert ist,
worin

| | |
|---|---|
| $R^7$ und $R^8$ | die oben angegebene Bedeutung haben, |
| | für Phenyl steht, das gegebenenfalls bis zu 2-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 4 Kohlenstoffatomen oder durch eine Gruppe der Formel $-NR^7R^8$ oder $-SO_2R^9$ substituiert ist, worin |
| $R^7$,$R^8$ und $R^9$ | die oben angegebene Bedeutung haben, |
| $R^3$ | für Wasserstoff oder für geradkettiges oder verzweigtes Alkyl mit bis zu 6 Kohlenstoffatomen oder Benzyl steht, oder |
| $R^2$ und $R^3$ | gemeinsam für den Rest der Formel $=CHR^{10}$ stehen, worin |
| $R^{10}$ | die oben angegebene Bedeutung von $R^5$ hat und mit dieser gleich oder verschieden ist, |
| D | für ein Sauerstoff- oder Schwefelatom oder für die $>$NH-Gruppe steht, |
| $R^4$ | für Wasserstoff oder für geradkettiges oder verzweigtes Alkyl mit bis zu 6 Kohlenstoffatomen oder Phenyl steht, wobei letztere gegebenenfalls bis zu 2-fach gleich oder verschieden durch Hydroxy, Halogen, Nitro, Cyano, Trifluormethyl, Trifluormethoxy, durch geradkettiges oder verzweigtes Alkoxy, Acyl oder Alkoxycarbonyl mit jeweils bis zu 4 Kohlenstoffatomen oder durch eine Gruppe der Formel $-NR^7R^8$ oder $-SO_2R^9$ substituiert sind, worin |
| $R^7$, $R^8$ und $R^9$ | die oben angegebene Bedeutung haben, oder für den Fall, daß D für die $>$NH-Gruppe steht, |
| $R^4$ | für die Gruppe der Formel $-SO_2R^9$ steht, worin |
| $R^9$ | die oben angegebene Bedeutung hat, |

gegebenenfalls in einer isomeren Form und deren physiologisch unbedenkliche Säureadditions-Salze und Metallsalzkomplexe bei der Bekämpfung der Dermatomykosen und Systemmykosen.

Besonders bevorzugt verwendet werden Verbindungen der allgemeinen Formel (I),
in welcher

| | |
|---|---|
| A und B | stets verschieden sind und für eine Gruppe der Formel $-CHR^5$ oder $-NR^6$ stehen, worin |
| $R^5$ | Wasserstoff oder geradkettiges oder verzweigtes Alkyl mit bis zu 4 Kohlenstoffatomen bedeutet, |
| $R^6$ | die unten angegebene Bedeutung von $R^2$ hat und mit dieser gleich oder verschieden ist, oder Methoxycarbonyl bedeutet, |
| $R^1$ | für Wasserstoff oder für geradkettiges oder verzweigtes Alkyl mit bis zu 4 Kohlenstoffatomen steht, |
| $R^2$ | für Wasserstoff oder für geradkettiges oder verzweigtes Alkyl mit bis zu 4 Kohlenstoffatomen steht, das gegebenenfalls 1- bis 2-fach, gleich oder verschieden durch Phenyl substituiert ist, das seinerseits durch Methoxy oder Ethoxy substituiert sein kann, oder für geradkettiges oder verzweigtes Acyl mit bis zu 4 Kohlenstoffatomen, oder für tert. Butyloxycarbonyl, oder für eine Gruppe der Formel $-SO_2R^9$ steht, worin |
| $R^9$ | geradkettiges oder verzweigtes Alkyl mit bis zu 4 Kohlenstoffatomen, Phenyl oder Benzyl bedeutet, wobei letztere gegebenenfalls durch Hydroxy, Fluor, Chlor, Brom, Nitro, Cyano, Methyl, Ethyl oder Methoxy substituiert sind, |
| $R^3$ | für Wasserstoff oder für geradkettiges oder verzweigtes Alkyl mit bis zu 4 Kohlenstoffatomen oder für Benzyl steht, oder |

$R^2$ und $R^3$ gemeinsam für den Rest der Formel $=CHR^{10}$ stehen, worin

$R^{10}$ die oben angegebene Bedeutung von $R^5$ hat und mit dieser gleich oder verschieden ist,

D für ein Sauerstoff- oder ein Schwefelatom oder für die $>$NH-Gruppe steht,

$R^4$ für Wasserstoff oder für geradkettiges oder verzweigtes Alkyl mit bis zu 4 Kohlenstoffatomen oder Phenyl steht, wobei letztere gegebenenfalls durch Fluor, Chlor, Brom, Nitro, Cyano, Methoxy, Ethoxy oder durch eine Gruppe der Formel $-NR^7R^8$ oder $-SO_2R^9$ substituiert sind, worin

$R^7$ und $R^8$ gleich oder verschieden sind und Wasserstoff, Methyl oder Ethyl bedeuten und

$R^9$ die oben angegebene Bedeutung hat, oder im Fall, daß D für die $>$NH-Gruppe steht,

$R^4$ für die Gruppe der Formel $-SO_2R^9$ steht, worin

$R^9$ die oben angegebene Bedeutung hat,

gegebenenfalls in einer isomeren Form, und deren physiologisch unbedenkliche Säureadditions-Salze und Metallsalzkomplexe bei der Bekämpfüng der Dermatomykosen und Systemmykosen.

Ganz besonders bevorzugt verwendet werden Verbindungen der allgemeinen Formel (I), in welcher die beiden Substituenten $-NR^2R^3$ und $-CO-D-R^4$ in der cis-Stellung vorliegen bei der Bekämpfung der Dermatomykosen und Systemmykosen.

Die Erfindung betrifft außerdem neue Wirkstoffe der allgemeinen Formel (Ia)

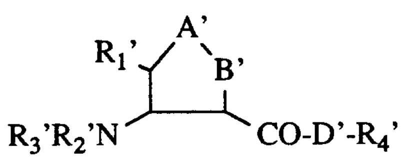

(Ia),

in welcher

A' und B' stets verschieden sind und für die Gruppe der Formel $-CHR^{5'}$ oder $-NR^{6'}$ stehen, worin

$R^{5'}$ Wasserstoff oder geradkettiges oder verzweigtes Alkyl mit bis zu 8 Kohlenstoffatomen bedeutet, das gegebenenfalls durch Halogen, Hydroxy, Phenyl, Carboxy oder durch geradkettiges oder verzweigtes Alkoxy oder Alkoxycarbonyl mit jeweils bis zu 6 Kohlenstoffatomen substituiert ist,

$R^{6'}$ die unten angegebene Bedeutung von $R^{2'}$ hat und mit dieser gleich oder verschieden ist, oder Methoxycarbonyl bedeutet,

$R^{1'}$ für Wasserstoff oder für geradkettiges oder verzweigtes Alkyl mit bis zu 8 Kohlenstoffatomen steht, das gegebenenfalls 1- bis 2-fach gleich oder verschieden durch Halogen, Hydroxy, Phenyl, Carboxy oder durch geradkettiges oder verzweigtes Alkoxy, Acyl oder Alkoxycarbonyl mit jeweils bis zu 6 Kohlenstoffatomen oder durch eine Gruppe der Formel $-NR^{7'}R^{8'}$ substituiert ist, worin

$R^{7'}$ und $R^{8'}$ gleich oder verschieden sind und Wasserstoff, Phenyl oder geradkettiges oder verzweigtes Alkyl mit bis zu 6 Kohlenstoffatomen bedeuten,

$R^{2'}$ für Wasserstoff oder
für geradkettiges oder verzweigtes Alkyl mit bis zu 8 Kohlenstoffatomen steht, das gegebenenfalls 1- bis 2-fach gleich oder verschieden durch Hydroxy, Formyl oder durch geradkettiges oder verzweigtes Acyl mit bis zu 6 Kohlenstoffatomen oder durch Phenyl oder Benzoyl substituiert ist, die gegebenenfalls bis zu 2-fach gleich oder verschieden durch Halogen, Nitro, Cyano, oder durch geradkettiges oder verzweigtes Alkyl oder Alkoxy mit jeweils bis zu 6 Kohlenstoffatomen substituiert sind,
oder
für geradkettiges oder verzweigtes Acyl mit bis zu 8 Kohlenstoffatomen, oder
für Benzoyl steht, das gegebenenfalls wie oben beschrieben substituiert ist, oder

für eine Gruppe der Formel $-SO_2R^{9'}$ steht,
worin

$R^{9'}$ geradkettiges oder verzweigtes Alkyl mit bis zu 8 Kohlenstoffatomen, Benzyl oder Phenyl bedeutet, wobei letztere gegebenenfalls bis zu 3-fach gleich oder verschieden durch Halogen, Hydroxy, Nitro, Cyano, Trifluormethyl, Trifluormethoxy oder durch geradkettiges oder verzweigtes Alkyl, Alkoxy oder Alkoxycarbonyl mit jeweils bis zu 6 Kohlenstoffatomen, Carboxy oder durch die oben aufgeführte Gruppe $-NR^{7'}R^{8'}$ substituiert sind,
worin

$R^{7'}$ und $R^{8'}$ die oben angegebene Bedeutung haben,
für Phenyl steht, das gegebenenfalls bis zu 3-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 6 Kohlenstoffatomen oder durch eine Gruppe der Formel $-NR^{7'}R^{8'}$ oder $-SO_2R^{9'}$ substituiert ist,
worin

$R^{7'}$, $R^{8'}$ und $R^{9'}$ die oben angegebene Bedeutung haben,

$R^{3'}$ für Wasserstoff oder für geradkettiges oder verzweigtes Alkyl mit bis zu 8 Kohlenstoffatomen steht, das gegebenenfalls durch Phenyl substituiert ist,
oder

$R^{2'}$ und $R^{3'}$ gemeinsam für den Rest der Formel $=CHR^{10'}$ stehen,
worin

$R^{10'}$ die oben angegebene Bedeutung von $R^{5'}$ hat und mit dieser gleich oder verschieden ist,

D' für ein Sauerstoff- oder Schwefelatom oder für die >NH-Gruppe steht,

$R^{4'}$ für Wasserstoff oder für geradkettiges oder verzweigtes Alkyl mit bis zu 8 Kohlenstoffatomen oder Phenyl steht, wobei letztere gegebenenfalls bis zu 3-fach gleich oder verschieden durch Hydroxy, Halogen, Nitro, Cyano, Carboxy, Trifluormethyl, Trifluormethoxy, durch geradkettiges oder verzweigtes Alkoxy, im Fall von Phenyl auch durch Alkyl, Acyl oder Alkoxycarbonyl mit jeweils bis zu 6 Kohlenstoffatomen oder durch eine Gruppe der Formel $-NR^{7'}R^{8'}$ oder $-SO_2R^{9'}$ substituiert sind,
worin

$R^{7'}$, $R^{8'}$ und $R^{9'}$ die oben angegebene Bedeutung haben,
oder für den Fall, daß D für die >NH-Gruppe steht,

$R^{4'}$ für die Gruppe der Formel $-SO_2R^{9'}$ steht,
worin

$R^{9'}$ die oben angegebene Bedeutung hat,

mit der Maßgabe, daß wenn A' für die $-CH_2$-Gruppe, B' für die $-NR^{6'}$-Gruppe, D' für Sauerstoff und $R^{1'}$, $R^{2'}$, $R^{3'}$ und $R^{4'}$ für Wasserstoff stehen, $R^{6'}$ nicht für Wasserstoff stehen darf,
und im Fall, daß $R^{2'}$ für die tert.Butylgruppe und $R^{3'}$ für Wasserstoff steht, $R^{6'}$ nicht für die Acetylgruppe stehen darf,
und wenn B' für die $-CH_2$-Gruppe, $R^{1'}$, $R^{2'}$ und $R^{3'}$ für Wasserstoff, D' für Sauerstoff, $R^{4'}$ für Ethyl und A' für die $-NR^{6'}$-Gruppe stehen, $R^{6'}$ nicht Wasserstoff oder Benzoyl bedeuten darf.

Die erfindungsgemäßen Verbindungen der allgemeinen Formeln (I) und (Ia) können auch in Form ihrer Salze vorliegen. Im allgemeinen seien hier Salze mit organischen oder anorganischen Basen oder Säuren genannt.

Zu den Säuren, die addiert werden können, gehören vorzugsweise Halogenkohlenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Malonsäure, Oxalsäure, Gluconsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Physiologisch unbedenkliche Salze können ebenso Metall- oder Ammoniumsalze der erfindinngsgemäßen Verbindungen sein, welche eine freie Carboxylgruppe besitzen, sein. Besonders bevorzugt sind z.B. Natrium-, Kalium-, Magnesium oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexalamin, Dimethylaminoethanol, Arginin, Lysin oder Ethylendiamin.

Die erfindungsgemäßen Verbindungen der allgemeinen Formeln (I) und (Ia) können in stereoisomeren Formen existieren, beispielsweise entweder wie Bild und Spiegelbild (Enantiomere), oder die sich nicht wie

Bild und Spiegelbild (Diastereomere) verhalten, beziehungsweise als Diastereomerengemisch oder als reine cis- oder trans-Isomere vorliegen. Die Erfindung betrifft sowohl die Antipoden, Racemformen, Diastereomerengemische sowie die reinen Isomeren. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962]. Die Trennung in die stereoisomer einheitlichen Verbindungen erfolgt beispielsweise über eine chromatographische Racematspaltung von diastereomeren Estem und Amiden oder an optisch aktiven Phasen. Außerdem ist eine Kristallisation von diastereomeren Salzen möglich.

Die erfindungsgemäßen Verbindungen der allgemeinen Formel (I) und die neuen Verbindungen der allgemeinen Formel (Ia) und ihre Säureadditions-Salze und Metallsalzkomplexe weisen antimikrobielle, insbesondere starke antimykotische Wirkungen auf. Sie besitzen ein sehr breites antimykotisches Wirkspektrum, insbesondere gegen Dermatophyten und Sproßpilze sowie biphasische Pilze, z.B. gegen Candida-Arten wie Candida albicans, Epidermophyton-Arten wie Epidermophyton floccosum, Aspergillus-Arten wie Aspergillus niger und Aspergillus fumigatus, Trichophyton-Arten wie Trichophyton mentagrophytes, Microsporon-Arten wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:
Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Außerdem wurden Verfahren zur Herstellung der Verbindungen der allgemeinen Formeln (I) und (Ia) gefunden, dadurch gekennzeichnet, daß man

[A] im Fall, daß A und A' jeweils entsprechend für die $-NR^6$- oder $-NR^{6'}$-Gruppe und B bzw. B' entsprechend für die $-CHR^5$- oder $-CHR^{5'}$ stehen,
Verbindungen der allgemeinen Formel (II)

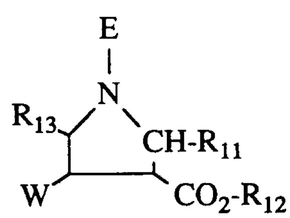

(II),

in welcher

| | |
|---|---|
| W | für eine Gruppe der Formel 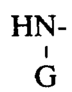 oder die $N_3$-Gruppe steht, |
| $R^{11}$ und $R^{13}$ | entsprechend den jeweiligen Bedeutungsumfang von $R^1/R^{1'}$ und $R^5/R^{5'}$ erfassen, |
| E | für einen $C_1$-$C_6$-Alkoxycarbonylrest steht, |
| G | für eine Aminoschutzgruppe, vorzugsweise für Benzyl steht, und |
| $R^{12}$ | für $C_1$-$C_6$-Alkylrest steht, |

zunächst nach üblichen Methoden durch Abspaltung der Schutzgruppe G, und im Falle, daß W für die Gruppe

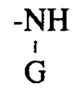

steht, und im Falle der $N_3$-Gruppe durch die Reaktion mit Triphenylphosphonoxid/$H_2O$, in die Verbindungen der allgemeinen Formel (III)

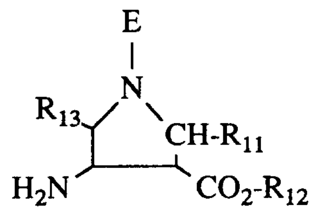

(III),

in welcher

$R^{11}$, $R^{12}$, $R^{13}$ und E die oben angegebene Bedeutung haben

überführt,

und anschließend mit aktivierten Carbonsäurederivaten, in Anwesenheit einer Base, zu den Verbindungen der allgemeinen Formel (IV)

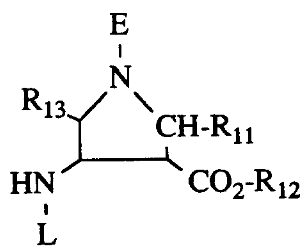

(IV),

in welcher

E, $R^{11}$, $R^{12}$ und $R^{13}$ die oben angegebene Bedeutung haben

und

L für einen $C_1$-$C_6$-Alkoxycarbonylrest steht,

umsetzt,

und in einem letzten Schritt durch Umsetzung mit HBr in Wasser gleichzeitig unter Abspaltung der Reste E, L und $R^{12}$ sowohl die Amin- als auch die Säurefunktion freisetzt,

oder Verbindungen der allgemeinen Formel (V)

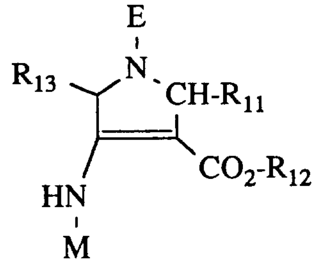

(V),

in welcher

E, $R^{11}$, $R^{12}$ und $R^{13}$ die oben angegebene Bedeutung haben

und

M mit Ausnahme von Wasserstoff die oben angegebene Bedeutung von $R^2$ hat, vorzugsweise aber für einen $(C_1-C_6)$-Acylrest, wie Acetyl, oder für den $-CH-(C_6H_4-OCH_3)_2$-Rest steht,

in inerten Lösemitteln durch Hydrierung mit Wasserstoff in Anwesenheit eines Katalysators oder mit Hydriden zu den entsprechenden Pyrrolidinverbindungen reduziert

oder

[B] im Fall, daß A und A' entsprechend für die $-CHR^5$ oder $CHR^{5'}$-Gruppe und B und B' entsprechend für die $-NR^6$- oder $-NR^{6'}$-Gruppe stehen,

Verbindungen der allgemeinen Formel (VI)

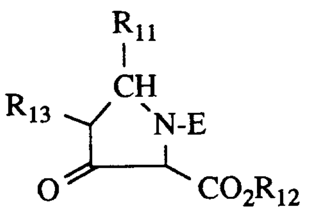

(VI),

in welcher

E, $R^{11}$, $R^{12}$ und $R^{13}$ die oben angegebene Bedeutung haben, mit Aminen der Formel (VII)

$NH_2-R^{14}$ (VII),

in welcher

$R^{14}$ für $(C_2-C_6)$-Alkyl oder Benzyl, vorzugsweise für Benzyl steht, in die entsprechenden Enaminoester der allgemeinen Formel (VIII)

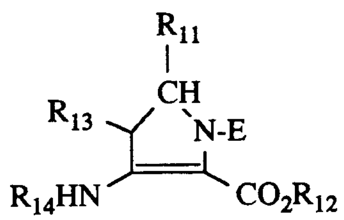

(VIII),

in welcher

E, $R^{11}$, $R^{12}$, $R^{13}$ und $R^{14}$ die oben angegebene Bedeutung haben,

überführt,

die Doppelbindung mit Wasserstoff in Anwesenheit eines Katalysators reduziert, und in einem letzten Schritt den Rest $R^{14}$ ebenfalls mit Wasserstoff in Anwesenheit eines Katalysators abspaltet

oder

direkt Verbindungen der allgemeinen Formel (VI) durch Umsetzung mit Ammoniak oder Ammoniumsalzen und anschließend unter Einwirkung von Wasserstoff in Anwesenheit eines Katalysators, oder mit Cyanoborhydrid, in Anwesenheit von Säuren in inerten Lösemitteln reduktiv aminiert,

und

im Fall, $R^2/R^3$ = H, die jeweiligen Schutzgruppen auf jeder der oben aufgeführten Stufen nach üblichen Bedingungen abspaltet,

und im Fall der Säuren [(I), (Ia) D = O, $R^4$ = H] gegebenenfalls die entsprechenden Ester verseift,

und im Fall, der für D, D', $R^4$ und $R^{4'}$ oben aufgeführten anderen Definitionen, ebenfalls nach üblichen Verfahren, wie beispielsweise Amidierung, Sulfonierung oder Sulfoamidierung, gegebenenfalls in Anwesenheit von Hilfsstoffen wie Katalysatoren und Dehydratisierungsmitteln, ausgehend von den entsprechenden Carbonsäuren, gegebenenfalls unter vorgeschalteter Aktivierung derivatisiert.

Die erfindungsgemäßen Verfahren können durch folgendes Formelschema beispielhaft erläutert werden:

[A]

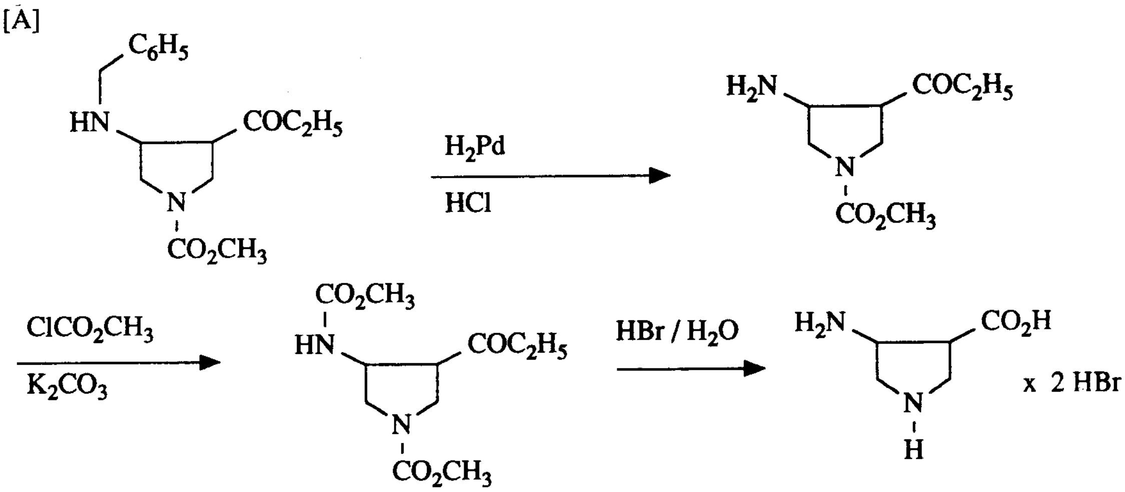


[A]

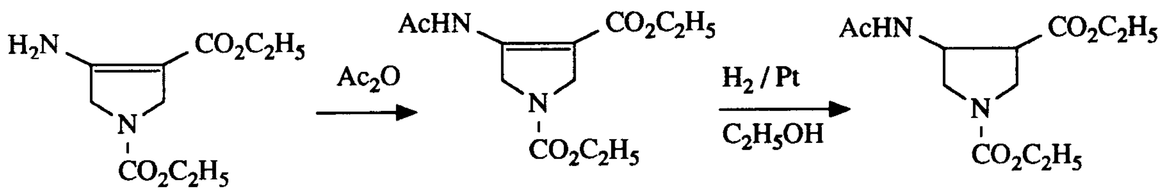

[A]

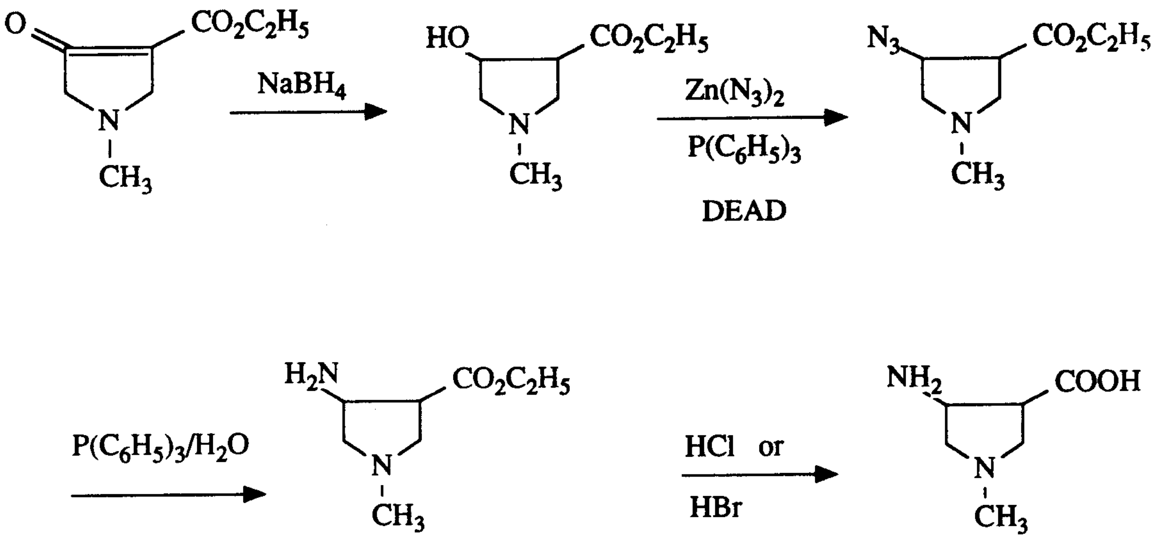


[B]

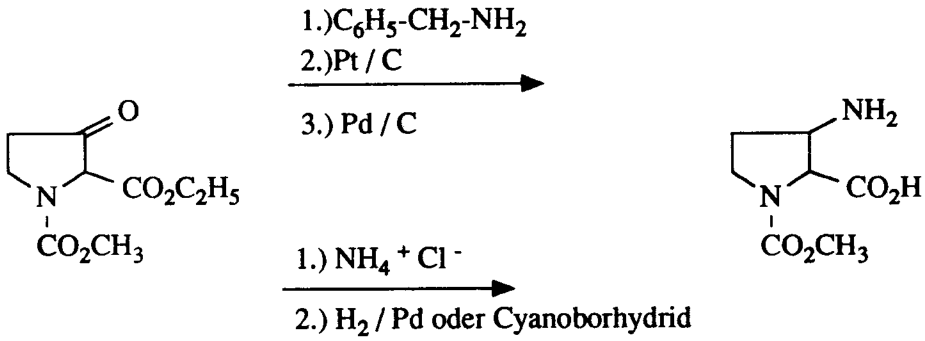


Aminoschutzgruppe im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert.-Butoxycarbonyl, Allyloxycarbonyl, Vinyloxycarbonyl, 2-Nitrobenzyloxycarbonyl, 3,4,5-Trimethoxybenzyloxycarbonyl, Cyclohexoxycarbonyl, 1,1-Dimethylethoxycarbonyl, Adamantylcarbonyl, Phthaloyl, 2,2,2-Trichlorethoxycarbonyl, 2,2,2-Trichlor-tertbutoxycarbonyl, Menthyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl oder 4-Nitrophenyl.

Als Lösemittel für sämtliche Schritte der Verfahren [A] und [B] kommen im allgemeinen Wasser und alle inerten organischen Lösemittel in Frage, die sich unter den Reaktionsbedingungen nicht verändern. Hierzu gehören vorzugsweise Alkohole wie Methanol, Ethanol, Propanol, Isopropanol, Ether wie Diethylether, Dioxan, Tetrahydrofuran, Glykolmonomethylether oder Glykoldimethylether, oder Amide wie Dimethylformamid, Dimethylacetamid oder Hexamethylphosphorsäuretriamid, oder Eisessig, Dimethylsulfoxid, Acetonitril oder Pyridin.

Als Basen eignen sich organische Amine (Trialkyl(C1-C6)amine) wie beispielsweise Triethylamin oder Heterocyclen wie Pyridin, Methylpiperidin, Piperidin oder Morpholin. Bevorzugt ist Triethylamin.

Die Reaktionstemperaturen können in einem größeren Breich varriiert werden. Im allgemeinen arbeitet man zwischen -78°C und +150°C, vorzugsweise zwischen -10°C und +100°C. Insbesondere bei der Siedetemperatur des jeweiligen Lösemittels.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Bei der Durchführung der erfindungsgemäßen Verfahrensvarianten [A] und [B] ist das Verhältnis der an der Reaktion beteiligten Stoffe beliebig. Im allgemeinen arbeitet man jedoch mit molaren Mengen der Reaktanden. Die Isolierung und Reinigung der erfindungsgmäßen Substanzen erfolgt vorzugsweise derart, daß man das Lösemittel im Vakuum abdestilliert und den gegebenenfalls erst nach Eiskühlung kristallin erhaltenen Rückstand aus einem geeigneten Lösemittel umkristallisiert. In einigen Fällen kann es erforderlich sein, die erfindungsgemäßen Verbindungen durch Chromatographie zu reinigen.

Die Abspaltung der Aminoschutzgruppen erfolgt in an sich bekannter Weise [vgl. Th. Greene, "Protective Groups in Organic Synthesis", 1. Aufl. J. Wiley & Sons, New York 1981 und Houben-Weyl "Methoden der organischen Chemie", Band XV/1 und 1].

Die Hydrierungen (Reduktionen, Abspaltungen von Schutzgruppen) erfolgen im allgemeinen in einem der oben aufgeführten Lösemitteln wie Alkoholen, beispielsweise Methanol, Ethanol oder Propanol, in Anwesenheit eines Edelmetallkatalysators wie Platin, Palladium, Palladium auf Tierkohle oder Raney-Nickel, im Fall der Doppelbindung der allgemeinen Formel (V) bevorzugt mit $H_2$/Platin, im Fall der reduktiven Aminierung der Verbindungen der allgemeinen Formel (VI) und der Verbindung der Enaminoester der allgemeinen Formel (VIII) bevorzugt mit $H_2$/Palladium. Bei der reduktiven Aminierung bzw. Hydrierung der Verbindungen der allgemeinen Formeln (V) und (VI) ist es ebenso möglich, Hydride, wie beispielsweise komplexe Borhydride oder Aluminiumhydride einzusetzen. Bevorzugt werden hierbei Natriumborhydrid oder Natriumcyanoborhydrid eingesetzt. In diesem Fall wird im allgemeinen in einem Temperaturbereich von -10°C bis +30°C, vorzugsweise bei Raumtemperatur, gearbeitet.

Als Katalysatoren werden im allgemeinen Säuren verwendet. Hierzu gehören bevorzugt anorganische Säuren wie beispielsweise Salzsäure oder Schwefelsäure, oder organische Sulfon- oder Carbonsäuren wie beispielsweise Methansulfonsäure, Ethansulfonsäure, Benzolsulfonsäure, Toluolsulfonsäure, Essigsäure oder Propionsäure.

Die Hydrierungen können bei normalem, erhöhtem oder erniedrigtem Druck (z.B. 0,5 - 5 bar) durchgeführt werden.

Die Katalysatoren und Basen werden im allgemeinen in einer Menge von 0 mol bis 10 mol, bevorzugt von 1,5 mol bis 3,5 mol, jeweils bezogen auf 1 mol der Verbindungen der allgemeinen Formeln (IV), (V), (VI) und (VIII), eingesetzt.

Die Säure wird im allgemeinen in einer Menge von 2 mol bis 30 mol, bevorzugt von 5 mol bis 15 mol, jeweils bezogen auf 1 mol der Verbindungen der allgemeinen Formeln (IV), (V), (VI) und (VIII), eingesetzt.

Die Verseifung der Carbonsäureester erfolgt nach üblichen Methoden, indem man die Ester in inerten Lösemitteln mit üblichen Basen behandelt, wobei die zunächst entstehenden Salze durch Behandeln mit Säure in die freien Carbonsäuren überführt werden können.

Die Verseifung der Carbonsäureester kann ebenso mit einer der oben aufgeführten Säuren durchgeführt werden.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliumethanolat, Kaliummethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet.

Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base oder die Säure im allgemeinen in einer Menge von 1 bis 3 mol, bevorzugt von 1 bis 1,5 mol, bezogen auf 1 mol des Esters, eingesetzt. Besonders bevorzugt

verwendet man molare Mengen der Reaktanden.

Bei der Durchführung der Reaktion entstehen im ersten Schritt die Salze der erfindungsgemäßen Verbindungen als Zwischenprodukte, die isoliert werden können. Die erfindungsgemäßen Säuren erhält man durch Behandeln der Salze mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren als vorteilhaft erwiesen, die basische Reaktionsmischung der Verseifung in einem zweiten Schritt ohne Isolierung der Salze anzusäuern. Die Säuren können dann in üblicher Weise isoliert werden.

Beispielhaft für die oben aufgeführten Derivatisierungsmöglichkeiten sollen hier die Amidierung und Sulfonierung bzw. Sulfonamidierung erläutert werden.

Die Amidierung erfolgt im allgemeinen in inerten Lösemitteln in Anwesenheit einer Base und eines Dehydratisierungsmittels.

Als Lösemittel eignen sich hierbei inerte organische Lösemittel, die sich unter den Reaktionsbedingungen nicht verändern. Hierzu gehören Halogenkohlenwasserstoffe wie Dichlormethan, Trichiormethan, Tetrachlormethan, 1,2-Dichlorethan, Trichlorethan, Tetrachlorethan, 1,2-Dichlorethan oder Trichiorethylen, Kohlenwasserstoffe wie Benzol, Xylol, Toluol, Hexan, Cyclohexan, oder Erdölfraktionen, Nitromethan, Dimethylformamid, Acetonitril oder Hexamethylphosphorsäuretriamid. Ebenso ist es möglich, Gemische der Lösemittel einzusetzen. Besonders bevorzugt ist Dichlormethan.

Als Basen für die Amidierung eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.buylat, oder organische Amine wie Benzyltrimethylammoniumhydroxid, Tetrabutylammoniumhydroxid, Pyridin, Triethylamin oder N-Methylpiperidin.

Die Amidierung wird im allgemeinen in einem Temperaturbereich von 0°C bis 150°C, bevorzugt bei 25°C bis 40°C, durchgeführt.

Die Amidierung wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid [vgl. J.C. Sheehan, S.L. Ledis, J. Am. Chem. Soc. 95, 875(1973); F.E. Freeman et al., J. Biol. Chem. 225, 507 (1982) und N.B. Benoton, K. Kluroda, Int. Pept. Prot. Res. 13, 403 (1979), 17, 187(1981)].

Die Sulfonierung oder Sulfoamidierung erfolgt in den oben erwähnten inerten Lösemitteln, gegebenenfalls unter Einsatz der ebenfalls oben aufgeführten Basen und Dehydratisierungsmittel.

Die Sulfonierung und Sulfoamidierung werden im allgemeinen bei Normaldruck durchgeführt. Es ist aber auch möglich, die Verfahren bei Unterdruck oder Überdruck durchzuführen (z.B. in einem Bereich von 0,5 bis 5 bar).

Die Sulfonierung und die Sulfoamidierung wird im allgemeinen in einem Temperaturbereich von 0°C bis +150°C, bevorzugt von +25°C bis +40°C, durchgeführt.

Zur Amidierung eignen sich im allgemeinen die literaturbekannten, käuflichen Amine und deren Derivate [vgl. Houben-Weyl, "Methoden der organischen Chemie", Bad. XI/1 und XI/2].

Die Sulfonierung und Sulfoamidierung erfolgen im allgemeinen ebenfalls mit den üblichen Sulfonsäuren und deren aktivierte Derivate [vgl. Houben-Weyl, "Methoden der organischen Chemie", Band IX, S 407 ff; Beilstein 11, 26].

Die Veresterung der Säuren erfolgt nach üblicher Methode, indem man die Säuren gegebenenfalls in einem der oben aufgeführten Lösemittel in Anwesenheit eines Katalysators mit den entsprechenden Alkoholen umsetzt. Bevorzugt wird dieser Alkohol auch als Lösemittel eingesetzt.

Als Katalysatoren können anorganische Säuren, wie beispielsweise Schwefelsäure oder anorganische Säurechloride, wie beispielsweise Thionylchlorid, eingesetzt werden.

Im allgemeinen setzt man 0,01 bis 1, bevorzugt 0,05 bis 0,5 mol Katalysator, bezogen auf 1 mol Reaktionspartner, ein.

Sowohl die Veresterung als auch die Amidierung können gegebenenfalls über aktivierte Stufen der Carbonsäuren, wie beispielsweise Säurehalogenide, die aus der entsprechenden Säure durch Umsetzung

mit Thionylchlorid, Phosphortrichlorid, Phosphorpentachlorid, Phosphortribromid oder Oxalylchlorid hergestellt werden können, verlaufen.

Die Säureadditions-Salze der Verbindungen der Formeln (I) und (Ia) können in einfacher Weise nach üblichen Salzbildungsmethoden, z.B. durch Lösen einer Verbindung der Formel (I) oder (Ia) in einem geeigneten Lösemittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösemittel gereinigt werden.

Die Verbindungen der allgemeinen Formeln (II), (III), (IV) und (V) sind teilweise bekannt [vgl. Acta Chemica Scandinavica B. 35 (1981), 473-479] oder neu und können dann aber in Analogie zu denen in der oben zitierten Literaturstelle publizierten Methoden hergestellt werden.

Die Verbindungen der allgemeinen Formel (VI) sind ebenfalls teilweise bekannt [vgl. J. Am. Chem. Soc. Vol. 86, 1964, 5293-5298] oder neu und können dann nach dem dort aufgeführten Verfahren hergestellt werden.

Die Amine der allgemeinen Formel (VII) sind bekannt [vgl. z.B. Beilstein 12, 1013].

Die Enaminoester der allgemeinen Formel (VIII) sind teilweise bekannt oder neu und können dann nach dem unter [B] beschriebenen Verfahren beispielsweise hergestellt werden.

Die vorstehenden Herstellungsverfahren sind lediglich zur Verdeutlichung angegeben. Die Herstellung der erfindungsgemäßen Verbindungen der allgemeinen Formeln (I) und (Ia) ist nicht auf diese Verfahren beschränkt, jede Modifikation dieser Verfahren ist in gleicher Weise für die Herstellung anwendbar.

Die erfindungsgemäßen Verbindungen der allgemeinen Formeln (I) und (Ia) und ihre Säureadditions-Salze weisen antimikrobielle, insbesondere starke antimykotische Wirkungen auf. Sie besitzen ein sehr breites antimykotisches Wirkspektrum, insbesondere gegen Dermatophyten und Sproßpilze sowie biphasische Pilze, z.B. gegen Candida-Arten wie Candida albicans, Epidermophyton-Arten wie Epidermophyton floccosum, Aspergillus-Arten wie Aspergillus niger und Aspergillus fumigatus, Trichophyton-Arten wie Trichophyton mentagrophytes, Microsporon-Arten wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:
Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiete in der Tiermedizin können beispielsweise aufgeführt werden: Alle Dermatomykosen und Systemmykosen, insbesondere solche, die durch die oben genannten Erreger hervorgerufen werden.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenne Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise etwa 0,01 bis 5 mg/kg Körpergewicht zur Erzielung wirksamer Ergebnisse zu verabreichen, und bei oraler Applikation beträgt die Dosierung etwa 0,01 bis 25 mg/kg, vorzugsweise 0,1 bis 10 mg/kg Köpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannnte obere Grenze überschrittten werden muß. Im Falle der Applikation größerer Mengen kann es empfehlenswert sein, diese

in mehreren Einzelgaben über den Tag zu verteilen.

Ausgangsverbindungen

Beispiel I

1-Methoxycarbonyl-4-(4,4'-dimethoxybenzhydryl)-amino-3,4-dehydropyrrolidin-3-carbonsäuremethylester

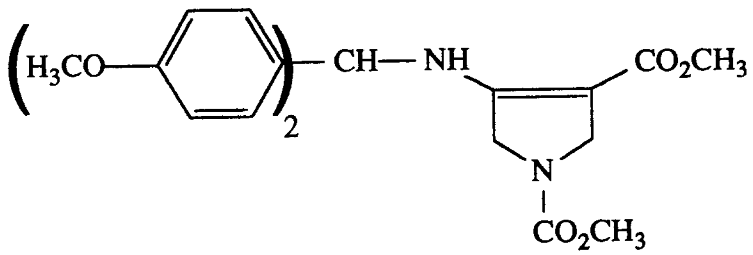

Die Darstellung erfolgt nach Standardmethode aus 1-Methoxycarbonyl-4-oxopyrrolidin-3-carbonsäuremethylester und 4,4'-Dimethoxybenzhydrylamin.

Beispiel II

4-(4,4'-Dimethoxybenzhydryl)amino-1-methoxycarbonyl-3,4-dehydropyrrolidin-3-carbonsäureethylester

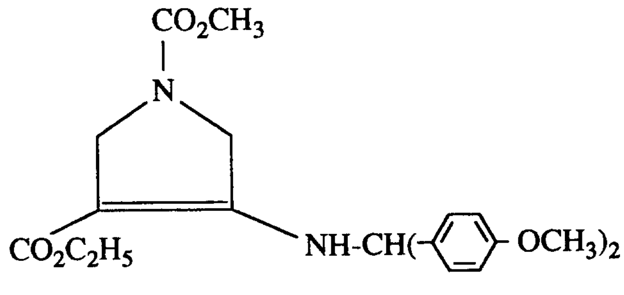

Beispiel II wird ausgehend von 1-Methoxycarbonyl-4-oxopyrrolidin-3-carbonsäureethylester und 4,4'-Dimethoxybenzhydrylamin darstellt (siehe Beispiel I).

Beispiel III

1-Methylpyrrolidin-3-on-4-carbonsäureethylester

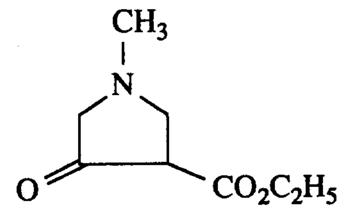

Ausgehend von N-(ß-Ethoxycarbonylethyl)-N-(ethoxycarbonylmethyl)methylaminwird die Verbindung durch eine Dieckmann-Cyclisierung erhalten.

Beispiel IV

1-Methoxycarbonylpyrrolidin-3-on-2-carbonsäureethylester

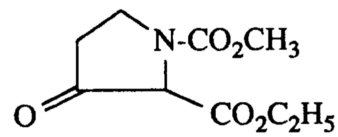

Die Synthese erfolgt in Analogie zu einem literaturbekannten Verfahren.

Beispiel V

4-(4,4'-Dimethoxybenzhydryl)amino-1-methyl-3,4-dehydropyrrolidin-3-carbonsäureethylester

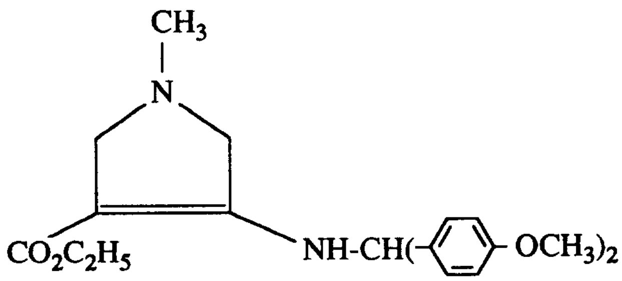

Eine Lösung von 21,5 g (0,125 mol) 1-Methylpyrrolidin-3-on-4-carbonsäureethylester, 30,3 g (0,125 mol) Bis(p-methoxyphenyl)methylamin und 0,4 g p-Toluolsulfonsäure in 400 ml Benzol wird am Wasserabscheider 16 h unter Rückfluß gehalten. Nach Einengen der Reaktionsmischung erhält man 49,5 g (100 %) eines orangen Öls.

$^1$H-NMR (200 MHz, $CDCl_3$):

$\delta$ = 1,24 (t, 3H); 2,38 (s, 3H); 3,45-3,60 (m, 4H); 3,77 (s, 6H); 4,13 (q, 2H); 5,42 (d, 1H); 6,83 (m, 4H); 7,16 (m, 4H); 7,61 (d, 1H).

MS (Cl, Isobutan:

m/e: 396 [$M^+$]

Beispiel VI

4-Hydroxy-1-methoxycarbonylpyrrolidin-3-carbonsäureethylester

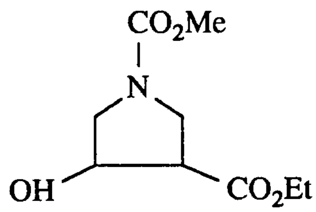

10 g (46,5 mmol) 1-Methoxycarbonylpyrrolidin-3-on-4-carbonsäureethylester in 100 ml Ethanol wurden über 20 min bei 0 °C mit 0,95 g (25 mmol) Natriumborhydrid reduziert. Die Reaktionsmischung wurde mit $H_2SO_4$ angesäuert, mit Wasser verdünnt und mehrfach mit Ether extrahiert. Nach Trocknung werden die vereinigten etherischen Phasen zu einem farblosen Öl eingeengt (4,7 g, 47 %).
MS (Cl, Isobutane):
m/e: 217 [$M^+$].

Herstellungsbeispiele

Beispiel 1

1-Methoxycarbonyl-4-(4,4'-dimethoxyberzhydryl)-aminopyrrolidin-3-carbonsäuremethylester

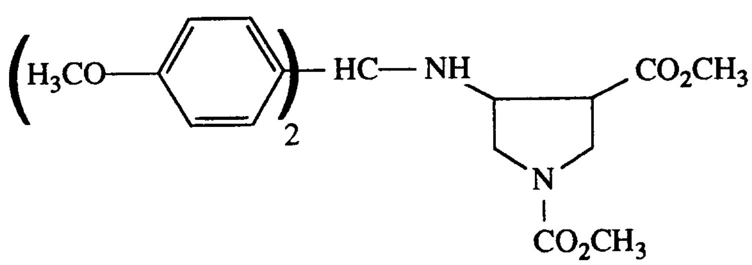

6 g (14 mmol) 1-Methoxycarbonyl-4-(4,4'-dimethoxybenzhydryl)-amino-3,4-dehydropyrrolidin-3-carbonsäuremethylester werden in 90 ml Ethanol bei Raumtemperatur und pH 3-4 12 h mit 0,7 g (14 mmol) Natriumcyanoborhydrid reduziert. Der nach dem Einengen erhaltene Rückstand wird in Ethylacetat aufgenommen, mit 1 N Natriumhydrogencarbonat-Lösung und Wasser gewaschen und getrocknet. Nach Trocknen und Einengen wird das erhaltene Öl mit Petrolether-Ethylacetat (1:1) an Florisil chromatographiert. Es werden 2,3 g (38 % der Theorie) eines hellen, hochviskosen Öls erhalten.
$^1$H-NMR (200 MHz, $CDCl_3$):
$\delta$ = 2,95-3,60 (m, 6H); 3,69 (s, 3H); 3,70 (s, 3H); 3,78 (s, 3H); 3,80 (s, 3H); 4,75 (s, br, 1H); 6,78-6,90 und 7,20-7,30 (m, 8H).

Beispiel 2

1-Methoxycarbonyl-4-aminopyrrolidin-3-carbonsäuremethylester

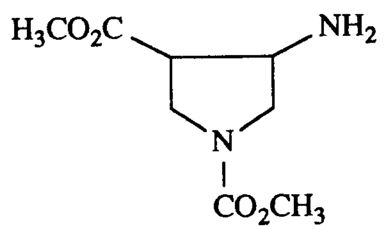

Eine Lösung von 2,3 g (5 mmol) 1-Methoxycarbonyl-4-(4,4'-dimethoxybenzhydryl)-aminopyrrolidin-3-carbonsäuremethylester und 5 ml Eisessig-Wasser (1:1) wird 5 min bei 100 °C gerührt. Nach dem Abkühlen verdünnt man mit ca. 50 ml Wasser, extrahiert 2mal mit Diethylether und engt dann die wäßrige Phase auf ca. 15-20 ml ein. Bei 0 °C wird die wäßrige Phase mit konz. Ammoniaklösung auf pH 10 gebracht und nach Sättigen mit Kochsalz mehrmals mit Diethylether extrahiert. Nach dem Trocknen und Einengen werden 0,35 g (35 % der Theorie) eines leicht gelben Öls erhalten.
MS (Cl, Isobutan):
m/z: 202 $[M^+]$

Beispiel 3

4-(4,4'-Dimethoxybenzhydryl)amino-1-methoxycarbonylpyrrolidin-3-carbonsäureethylester

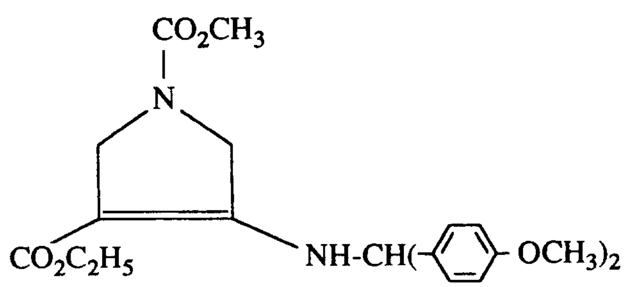

10 g (22,7 mmol) 4-(4,4'-Dimethoxybenzhydryl)amino-1-methoxycarbonyl-3,4-dehydropyrrolidin-3-carbonsäureethylester in 150 ml Ethanol werden mit 1,43 g (22,7 mmol) Natriumcyanoborhydrid über 24 h bei Raumtemperatur und pH 3-4 reduziert. Nach Einengen der Reaktionslösung wird der Rückstand in Ethylacetat aufgenommen, mit Natriumhydrogencarbonatlösung und Wasser gewaschen und getrocknet. Das Lösungsmittel wird im Vakuum abgezogen und der Rückstand an Florisil mit Petrolether/Essigester (1:1) chromatographiert. Es werden 4,2 g (41,9 %) eines viskosen Öls erhalten.
MS (Cl, Isobutan):
m/e: 442 $[M^+]$.

Beispiel 4

4-Benzylamino-1-methoxycarbonylpyrrolidin-3-carbonsäureethylester

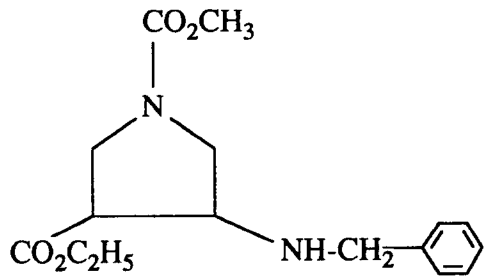

96 g (0,316 mol) 4-Benzylamino-1-methoxycarbonyl-3,4-dehydropyrrolidin-3-carbonsäureethylester in 1,6 l Ethanol werden über 24 h mit 22,8 g (0,362 mol) Natriumcyanoborhydrid bei Raumtemperatur und pH 3-4 reduziert. Nach Einengen der Reaktionsmischung wird der Rückstand in Ethylacetat aufgenommen, mit 1 N Natriumhydrogencarbonatlösung und Wasser gewaschen, anschließend getrocknet und eingeengt. Chromatographie an Florisil mit Petrolether und Essigester (1:2) liefert 81 g (83,8 %) eines gelblich viskosen Öls.
MS (Cl, Isobutan):
m/e: 306 [$M^+$].

Beispiel 5

4-(4,4'-Dimethoxybenzhydryl)amino-1-methylpyrrolidin-3-carbonsäureethylester

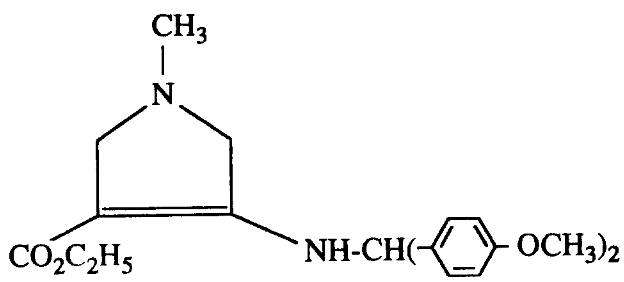

10 g (25,3 mmol) 4-(4,4'-Dimethoxybenzhydryl)amino-1-methyl-3,4-dehydropyrrolidin-3-carbonsäureethylester in 100 ml Ethanol werden bei Raumtemperatur und pH 3-4 über 24 h mit 1,76 g (28 mmol) Natriumcyanoborhydrid reduziert. Der nach Einengen erhaltene Rückstand wird in Essigester gelöst, mit Hydrogencarbonat-Lösung und Wasser gewaschen und anschließend getrocknet. Chromatographie an Florisil mit Petrolether/Essigester (1:1) liefert

Beispiel 6

4-tert-Butyloxycarbonylamino-1-methoxycarbonylpyrrolidin-3-carbonsäureethylester

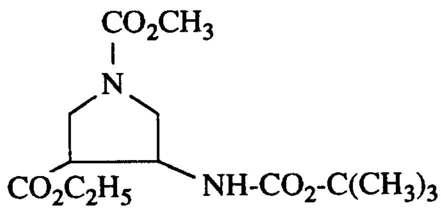

13,8 g (50 mmol) 4-Amino-1-methoxycarbonylpyrrolidin-3-carbonsäureethylester in 70 ml Dioxan werden mit 14,4 ml (102 mmol) Triethylamin und 13,9 ml (60,5 mmol) Di-tert-butyldicarbonat bei Raumtemperatur 16 h gerührt. Die Reaktionsmischung gibt man auf 250 ml Eiswasser und 100 ml Essigester, säuert mit $KHSO_4$-Lösung an und extrahiert mit Ethylacetat. Die vereinigten organischen Phasen werden mit $NaHCO_3$-Lösung gewaschen, getrocknet und eingeengt. Man erhält 6,9 g (45,7 %) eines leicht gelblichen Öls.
$^1$H-NMR (200 MHz, $CDCl_3$):
$\delta$ = 1,26 (t, 3H); 1,53 (s, 9H); 2,98-3,81 (m, 6H); 3,70 (s, 3H); 4,15 (q, 2H); 4,93 (d, 1H).

Beispiel 7

4-tert-Butyloxycarbonylamino-1-methoxycarbonylpyrrolidin-3-carbonsäure

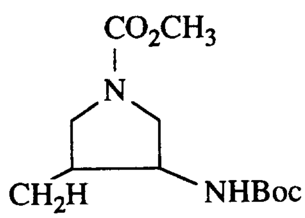

6,9 g (22 mmol) 4-tert-Butyloxycarbonylamino-1-methoxycarbonylpyrrolidin-3-carbonsäureethylester in 30 ml Methanol, 20 ml Tetrahydrofuran und 10 ml Wasser werden mit 1,5 g (62,5 mmol) Lithiumhydroxid 16 h bei Raumtemperatur gerührt. Die Reaktionsmischung wird mit $KHSO_4$-Lösung bei 0 °C angesäuert und mit Essigester extrahiert. Die vereinigten organischen Phasen liefern nach Waschen mit Wasser, Trocknen und Einengen 3,6 g (57 %) eines leicht gelblichen Öls.
$^1$H-NMR (200 MHz, DMSO-$d_6$):
$\delta$ = 1,40 (s, 9H); 2,85-4,2 (sev. m, 6H); 3,59 (s, 3H); 6,05 (d, 1H).
MS (CI, Isobutan):
m/e: 288 [$M^+$]

Beispiel 8

4-Amino-1-methoxycarbonylpyrrolidin-3-carbonsäure, Hydrochlorid

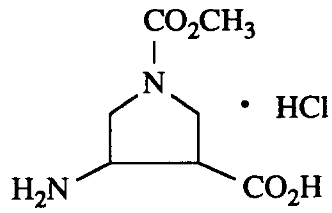

Eine Lösung von 1,5 g (5,2 mmol) 4-tert-Butyloxycarbonylamino-1-methoxycarbonylpyrrolidin-3-carbonsäure in 10 ml Dioxane wird mit 10 ml einer gesättigten HCl-Lösung in Dioxan versetzt und 4 h bei Raumtemperatur gerührt. Nach Einengen der Reaktionsmischung erhält man 1,15 g (98,5 %) hygroskopische Substanz.

$^1$H-NMR (200 MHz, $D_2O$):
$\delta$ = 3,32-3,99 (m, 6H); 3,71 (s, 3H).
MS (Cl, Isobutan):
m/e: 188 [$M^+$]

Beispiel 9

4-(N-Benzyl-N-tert-butoxycarbonylamino)-1-methoxycarbonylpyrrolidin-3-carbonsäureethylester

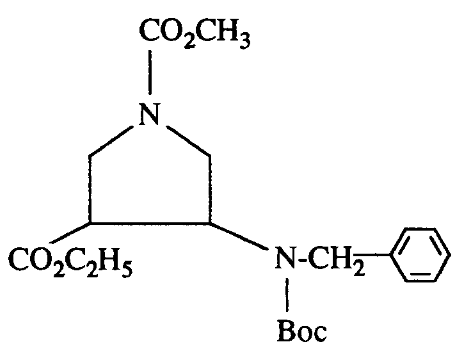

20 g (65,4 mmol) 4-Benzylamino-1-methoxycarbonylpyrrolidin-3-carbosäureethylester in 200 ml Dioxane werden mit 6,6 g (65,5 mmol) Triethylamin, 18,5 g (85 mmol) Di-tert-butyldicarbonat und 2 g Dimethylaminopyridin versetzt und bei Raumtemperatur 30 h gerührt. Nach Zugabe von 11 Wasser und 300 ml Ethylacetat wird mit Salzsäure auf pH 3-4 angesäuert und die wäßrige Phase mit mehr Ethylacetat extrahiert. Die vereinigten organischen Phasen werden mit Hydrogencarbonat- und Kochsalzlösung gewachen, getrocknet und eingeengt. Es werden 25,4 g (95,7 % eines hellgelben Öls erhalten.

$^1$H-NMR (200 MHz, $CDCl_3$):
$\delta$ = 1,24 (t, 3H); 1,50 (bs, 9H); 3,38-4,2 (m, 6H); 3,70 (s, 3H); 4,14 (q, 2H); 4,37 (m, 2H), 7,15-7,4 (m, 5H).

Beispiel 10

4-Amino-1-methoxycarbonylpyrrolidin-3-carbonsäureethylester

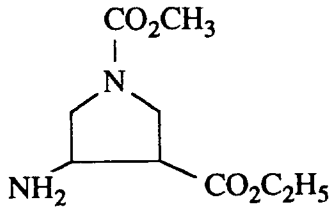

Eine Lösung von 15,0 g (50 mmol) 4-Benzylamino-1-methoxycarbonylpyrrolidin-3-carbonsäureethylester in 500 ml Ethanol, 500 ml Wasser und 250 ml 0,1 N Salzsäure wird in Gegenwart von Pd auf Kohle bei Raumtemperatur hydriert (10 bar $H_2$, 20 h). Man filtriert den Katalysator ab, engt das Filtrat ein und erhält so 7,6 g (79,2 %) eines hochviskosen Öls.

$^1$H-NMR (200 MHz, $CDCl_3$):

$\delta$ = 1,28 (t, 3H); 3,32-4,05 (m, 6H); 3,71 (s, 3H); 4,18 (q, 2H); 6,02 (bs, 2H).

Beispiel 11

4-Benzylamino-1-methoxycarbonylpyrrolidin-3-carbonsäure

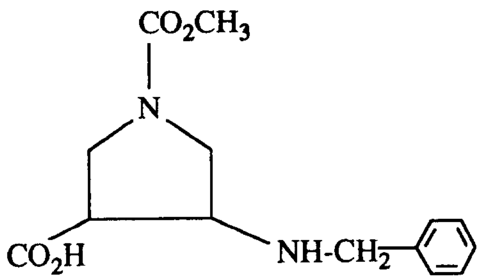

17 g (56 mmol) 4-Benzylamino-1-methoxycarbonylpyrrolidin-3-carbonsäureethylester und 4,8 g (200 mmol) Lithiumhydroxid in 50 ml Tetrahydrofuran, 10 ml Methanol und 10 ml Wasser werden 23 h bei Raumtemperatur gerührt. Die Reaktionsmischung wird mit ca. 155 ml 1 N $KHSO_4$-Lösung neutralisiert und mehrfach mit Diethylether extrahiert. Trocknen und Einengen der Ether-Phasen liefert 5,7 g (36,6 %) eines viskosen Öls.

$^1$H-NMR (200 MHz, $CDCl_3$):

$\delta$ = 2,61 (m, 1H); 3,30-3,85 (m, 6H); 3,72 (s, 3H); 4,25-4,41 (m, 2H); 7,20-7,40 (m, 5H).

Beispiel 12

4-Aminopyrrolidin-3-carbonsäure, Hydrobromid

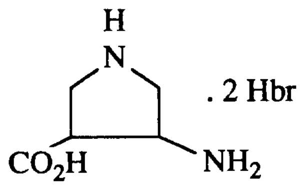

1,2 g (4,17 mmol) 4-tert-Butyloxycarbonylamino-1-methoxycarbonylpyrrolidin-3-carbonsäure in 10 ml Essigsäure werden mit 5 ml HBr-Eisessiglösung bei 60 ° C 2 h gerührt. Nach Abkühlen auf Raumtemperatur wird der entstandene Niederschlag abgesaugt. Man erhält 1,1 g (90 %) eines pinken, hygroskopischen Pulvers. $^1$H-NMR (200 MHz, $D_2O$):
$\delta$ = 3,47-4,03 (m, 5H); 4,29-4,50 (m, 1H).
MS (CI, Isobutan):
m/e: 130 [$M^+$]

Beispiel 13

4-Benzylamino-1-methoxycarbonyl-3,4-dehydropyrrolidin-3-carbonsäureethylester

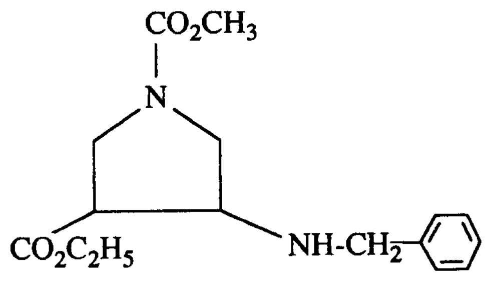

Analog zum Beispiel I wird die Verbindung aus 1-Methoxycarbonyl-4-oxopyrrolidin-3-carbonsäureethylester und Benzylamin hergestellt.

**Patentansprüche**

**1.** Substituierte Pyrrolidine der allgemeinen Formel (I)

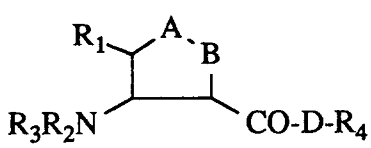

(I),

in welcher
A und B stets verschieden sind und
für eine Gruppe der Formel $-CHR^5$ oder $-NR^6$ stehen,
worin
$R^5$ Wasserstoff oder geradkettiges oder verzweigtes Alkyl mit bis zu 8 Kohlenstoff-

| | |
|---|---|
| | atomen bedeutet, das gegebenenfalls durch Halogen, Hydroxy, Phenyl, Carboxy oder durch geradkettiges oder verzweigtes Alkoxy oder Alkoxycarbonyl mit jeweils bis zu 6 Kohlenstoffatomen substituiert ist, |
| $R^6$ | die unten angegebene Bedeutung von $R^2$ hat und mit dieser gleich oder verschieden ist, oder Methoxycarbonyl bedeutet, |
| $R^1$ | für Wasserstoff oder für geradkettiges oder verzweigtes Alkyl mit bis zu 8 Kohlenstoffatomen steht, das gegebenenfalls 1- bis 2-fach gleich oder verschieden durch Halogen, Hydroxy, Phenyl, Carboxy oder durch geradkettiges oder verzweigtes Alkoxy, Acyl oder Alkoxycarbonyl mit jeweils bis zu 6 Kohlenstoffatomen oder durch eine Gruppe der Formel $-NR^7R^8$ substituiert ist, |
| | worin |
| $R^7$ und $R^8$ | gleich oder verschieden sind und Wasserstoff, Phenyl oder geradkettiges oder verzweigtes Alkyl mit bis zu 6 Kohlenstoffatomen bedeuten, |
| $R^2$ | für Wasserstoff oder |
| | für geradkettiges oder verzweigtes Alkyl mit bis zu 8 Kohlenstoffatomen steht, das gegebenenfalls 1- bis 2-fach gleich oder verschieden durch Hydroxy, Formyl oder durch geradkettiges oder verzweigtes Acyl mit bis zu 6 Kohlenstoffatomen oder durch Phenyl oder Benzoyl substituiert ist, die gegebenenfalls bis zu 2-fach gleich oder verschieden durch Halogen, Nitro, Cyano, oder durch geradkettiges oder verzweigtes Alkyl oder Alkoxy mit jeweils bis zu 6 Kohlenstoffatomen substituiert sind, |
| | oder |
| | für geradkettiges oder verzweigtes Acyl mit bis zu 8 Kohlenstoffatomen, oder |
| | für tert.Butyloxycarbonyl(Boc) oder Benzoyl steht, das gegebenenfalls wie oben beschrieben substituiert ist, oder |
| | für eine Gruppe der Formel $-SO_2R^9$ steht, |
| | worin |
| $R^9$ | geradkettiges oder verzweigtes Alkyl mit bis zu 8 Kohlenstoffatomen, Benzyl oder Phenyl bedeutet, wobei letztere gegebenenfalls bis zu 3-fach gleich oder verschieden durch Halogen, Hydroxy, Nitro, Cyano, Trifluormethyl, Trifluormethoxy oder durch geradkettiges oder verzweigtes Alkyl, Alkoxy oder Alkoxycarbonyl mit jeweils bis zu 6 Kohlenstoffatomen, Carboxy oder durch die oben aufgeführte Gruppe $-NR^7R^8$ substitituiert sind, |
| | worin |
| $R^7$ und $R^8$ | die oben angegebene Bedeutung haben, |
| | für Phenyl steht, das gegebenenfalls bis zu 3-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 6 Kohlenstoffatomen oder durch eine Gruppe der Formel $-NR^7R^8$ oder $-SO_2R^9$ substituiert ist, |
| | worin |
| $R^7$, $R^8$ und $R^9$ | die oben angegebene Bedeutung haben, |
| $R^3$ | für Wasserstoff oder für geradkettiges oder verzweigtes Alkyl mit bis zu 8 Kohlenstoffatomen steht, das gegebenenfalls durch Phenyl substituiert ist, |
| | oder |
| $R^2$ und $R^3$ | gemeinsam für den Rest der Formel $=CHR^{10}$ stehen, |
| | worin |
| $R^{10}$ | die oben angegebene Bedeutung von $R^5$ hat und mit dieser gleich oder verschieden ist, |
| D | für ein Sauerstoff- oder Schwefelatom oder für die $>$NH-Gruppe steht, |
| $R^4$ | für Wasserstoff oder für geradkettiges oder verzweigtes Alkyl mit bis zu 8 Kohlenstoffatomen oder Phenyl steht, wobei letztere gegebenenfalls bis zu 3-fach gleich oder verschieden durch Hydroxy, Halogen, Nitro, Cyano, Carboxy, Trifluoromethyl, Trifluormethoxy, durch geradkettiges oder verzweigtes Alkoxy, im Fall von Phenyl auch durch Alkyl, Acyl oder Alkoxycarbonyl mit jeweils bis zu 6 Kohlenstoffatomen oder durch eine Gruppe der Formel $-NR^7R^8$ oder $-SO_2R^9$ substituiert sind, |
| | worin |
| $R^7$, $R^8$ und $R^9$ | die oben angegebene Bedeutung haben, |

oder für den Fall, daß D für die $>$NH-Gruppe steht,
$R^4$ für die Gruppe der Formel $-SO_2R^9$ steht,
worin
$R^9$ die oben angegebene Bedeutung hat,
zur Bekämpfung von Krankheiten.

**2.** Substituierte Pyrrolidine der allgemeinen Formel (I) gemäß Anspruch 1, in welcher
A und B stets verschieden sind und
für eine Gruppe der Formel $-CHR^5$ oder $-NR^6$ stehen,
worin
$R^5$ Wasserstoff oder geradkettiges oder verzweigtes Alkyl mit bis zu 6 Kohlenstoffatomen bedeutet, das gegebenenfalls durch Halogen, Hydroxy, oder durch geradkettiges oder verzweigtes Alkoxy oder Alkoxycarbonyl mit jeweils bis zu 4 Kohlenstoffatomen substituiert ist,
$R^6$ die unten angegebene Bedeutung von $R^2$ hat und mit dieser gleich oder verschieden ist, oder Methoxycarbonyl bedeutet,
$R^1$ für Wasserstoff oder für geradkettiges oder verzweigtes Alkyl mit bis zu 6 Kohlertstoffatomen steht, das gegebenenfalls durch Halogen, Hydroxy, durch geradkettiges oder verzweigtes Alkoxy, Acyl oder Alkoxycarbonyl mit jeweils bis zu 4 Kohlenstoffatomen oder durch eine Gruppe der Formel $-NR^7R^8$ substituiert ist,
worin
$R^7$ und $R^8$ gleich oder verschieden sind und Wasserstoff oder geradkettiges oder verzweigtes Alkyl mit bis zu 4 Kohlenstoffatomen bedeuten,
$R^2$ für Wasserstoff oder
für geradkettiges oder verzweigtes Alkyl mit bis zu 6 Kohlenstoffatomen steht, das gegebenenfalls 1 - bis 2-fach, gleich oder verschieden durch Hydroxy, Formyl oder durch geradkettiges oder verzweigtes Acyl mit bis zu 4 Kohlenstoffatomen oder durch Phenyl oder Benzoyl substiutiert ist, die gegebenenfalls durch Halogen, Nitro, Cyano, oder durch geradkettiges oder verzweigtes Alkyl oder Alkoxy mit jeweils bis zu 4 Kohlenstoffatomen substituiert sind,
oder
für geradkettiges oder verzweigtes Acyl mit bis zu 6 Kohlenstoffatomen oder für tert. Butyloxycarbonyl(Boc) oder Benzoyl steht, das gegebenenfalls wie oben beschrieben substituiert ist,
oder
für eine Gruppe der Formel $-SO_2R^9$ steht,
worin
$R^9$ geradkettiges oder verzweigtes Alkyl mit bis zu 6 Kohlenstoffatomen, Phenyl oder Benzyl bedeutet, wobei letztere gegebenenfalls bis zu 2-fach gleich oder verschieden durch Halogen, Hydroxy, Nitro, Cyano, Trifluormethyl, Trifluormethoxy oder durch geradkettiges oder verzweigtes Alkyl oder Alkoxy mit jeweils bis zu 4 Kohlenstoffatomen oder durch die oben aufgeführte Gruppe der Formel $-NR^7R^8$ substituiert ist,
worin
$R^7$ und $R^8$ die oben angegebene Bedeutung haben,
für Phenyl steht, das gegebenenfalls bis zu 2-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 4 Kohlenstoffatomen oder durch eine Gruppe der Formel $-NR^7R^8$ oder $-SO_2R^9$ substituiert ist,
worin
$R^7$, $R^8$ und $R^9$ die oben angegebene Bedeutung haben,
$R^3$ für Wasserstoff oder für geradkettiges oder verzweigtes Alkyl mit bis zu 6 Kohlenstoffatomen oder Benzyl steht,
oder
$R^2$ und $R^3$ gemeinsam für den Rest der Formel $=CHR^{10}$ stehen,
worin
$R^{10}$ die oben angegebene Bedeutung von $R^5$ hat und mit dieser gleich oder verschieden ist,

D für ein Sauerstoff- oder Schwefelatom oder für die >NH-Gruppe steht,

$R^4$ für Wasserstoff oder für geradkettiges oder verzweigtes Alkyl mit bis zu 6 Kohlenstoffatomen oder Phenyl steht, wobei letztere gegebenenfalls bis zu 2-fach gleich oder verschieden durch Hydroxy, Halogen, Nitro, Cyano, Trifluormethyl, Trifluormethoxy, durch geradkettiges oder verzweigtes Alkoxy, Acyl oder Alkoxycarbonyl mit jeweils bis zu 4 Kohlenstoffatomen oder durch eine Gruppe der Formel $-NR^7R^8$ oder $-SO_2R^9$ substituiert sind,

worin

$R^7$, $R^8$ und $R^9$ die oben angegebene Bedeutung haben,

oder für den Fall, daß D für die >NH-Gruppe steht,

$R^4$ für die Gruppe der Formel $-SO_2R^9$ steht,

worin

$R^9$ die oben angegebene Bedeutung hat,

zur Bekämpfung von Krankheiten.

**3.** Substituierte Verbindungen der allgemeinen Formel (I) gemäß Anspruch 1, in welcher

A und B stets verschieden sind und für eine Gruppe der Formel $-CHR^5$ oder $-NR^6$ stehen,

worin

$R^5$ Wasserstoff oder geradkettiges oder verzweigtes Alkyl mit bis zu 4 Kohlenstoffatomen bedeutet,

$R^6$ die unten angegebene Bedeutung von $R^2$ hat und mit dieser gleich oder verschieden ist, oder Methoxycarbonyl bedeutet,

$R^1$ für Wasserstoff oder für geradkettiges oder verzweigtes Alkyl mit bis zu 4 Kohlenstoffatomen steht,

$R^2$ für Wasserstoff oder für geradkettiges oder verzweigtes Alkyl mit bis zu 4 Kohlenstoffatomen steht, das gegebenenfalls 1- bis 2-fach, gleich oder verschieden durch Phenyl substituiert ist, das seinerseits durch Methoxy oder Ethoxy substituiert sein kann, oder für geradkettiges oder verzweigtes Acyl mit bis zu 4 Kohlenstoffatomen oder tert.Butyloxycarbonyl(Boc), oder für eine Gruppe der Formel $-SO_2R^9$ steht,

worin

$R^9$ geradkettiges oder verzweigtes Alkyl mit bis zu 4 Kohlenstoffatomen, Phenyl oder Benzyl bedeutet, wobei letztere gegebenenfalls durch Hydroxy, Fluor, Chlor, Brom, Nitro, Cyano, Methyl, Ethyl oder Methoxy substituiert sind,

$R^3$ für Wasserstoff oder für geradkettiges oder verzweigtes Alkyl mit bis zu 4 Kohlenstoffatomen oder Benzyl steht,

oder

$R^2$ und $R^3$ gemeinsam für den Rest der Formel $=CHR^{10}$ stehen,

worin

$R^{10}$ die oben angegebene Bedeutung von $R^5$ hat und mit dieser gleich oder verschieden ist,

D für ein Sauerstoff- oder ein Schwefelatom oder für die >NH-Gruppe steht,

$R^4$ für Wasserstoff oder für geradkettiges oder verzweigtes Alkyl mit bis zu 4 Kohlenstoffatomen oder Phenyl steht, wobei letztere gegebenenfalls durch Fluor, Chlor, Brom, Nitro, Cyano, Methoxy, Ethoxy oder durch eine Gruppe der Formel $-NR^7R^8$ oder $-SO_2R^9$ substituiert sind,

worin

$R^7$ und $R^8$ gleich oder verschieden sind und Wasserstoff, Methyl oder Ethyl bedeuten

und

$R^9$ die oben angegebene Bedeutung hat,

oder im Fall, daß D für die >NH-Gruppe steht,

$R^4$ für die Gruppe der Formel $-SO_2R^9$ steht,

worin

$R^9$ die oben angegebene Bedeutung hat,

zur Bekämpfung von Krankheiten.

**4.** Substituierte Verbindungen der allgemeinen Formel (I) gemäß Anspruch 1, in welcher die beiden Substituenten $-NR^2R^3$ und $-CO-D-R^4$, worin $R^2$, $R^3$, D und $R^4$ die in Anspruch 1 angegebene Bedeutung haben, in der cis-Stellung vorliegen, zur Bekämpfung von Dermatomykosen und Systemmykosen.

**5.** Verbindungen der allgemeinen Formel (Ia)

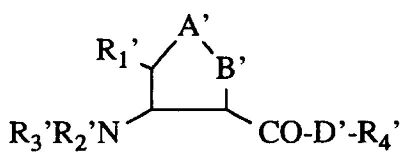

(Ia),

in welcher

| | |
|---|---|
| A' und B' | stets verschieden sind und für die Gruppe der Formel $-CHR^{5'}$ oder $-NR^{6'}$ stehen, worin |
| $R^{5'}$ | Wasserstoff oder geradkettiges oder verzweigtes Alkyl mit bis zu 8 Kohlenstoffatomen bedeutet, das gegebenenfalls durch Halogen, Hydroxy, Phenyl, Carboxy oder durch geradkettiges oder verzweigtes Alkoxy oder Alkoxycarbonyl mit jeweils bis zu 6 Kohlenstoffatomen substituiert ist, |
| $R^{6'}$ | die unten angegebene Bedeutung von $R^{2'}$ hat und mit dieser gleich oder verschieden ist, oder Methoxycarbonyl bedeutet, |
| $R^{1'}$ | für Wasserstoff oder für geradkettiges oder verzweigtes Alkyl mit bis zu 8 Kohlenstoffatomen steht, das gegebenenfalls 1- bis 2-fach gleich oder verschieden durch Halogen, Hydroxy, Phenyl, Carboxy oder durch geradkettiges oder verzweigtes Alkoxy, Acyl oder Alkoxycarbonyl mit jeweils bis zu 6 Kohlenstoffatomen oder durch eine Gruppe der Formel $-NR^{7'}R^{8'}$ substituiert ist, worin |
| $R^{7'}$ und $R^{8'}$ | gleich oder verschieden sind und Wasserstoff, Phenyl oder geradkettiges oder verzweigtes Alkyl mit bis zu 6 Kohlenstoffatomen bedeuten, |
| $R^{2'}$ | für Wasserstoff oder<br>für geradkettiges oder verzweigtes Alkyl mit bis zu 8 Kohlenstoffatomen steht, das gegebenenfalls 1- bis 2-fach gleich oder verschieden durch Hydroxy, Formyl oder durch geradkettiges oder verzweigtes Acyl mit bis zu 6 Kohlenstoffatomen oder durch Phenyl oder Benzoyl substituiert ist, die gegebenenfalls bis zu 2-fach gleich oder verschieden durch Halogen, Nitro, Cyano, oder durch geradkettiges oder verzweigtes Alkyl oder Alkoxy mit jeweils bis zu 6 Kohlenstoffatomen substituiert sind,<br>oder<br>für geradkettiges oder verzweigtes Acyl mit bis zu 8 Kohlenstoffatomen,<br>oder<br>für tert.Butyloxycarbonyl(Boc) oder Benzoyl steht, das gegebenenfalls wie oben beschrieben substituiert ist, oder<br>für eine Gruppe der Formel $-SO_2R^{9'}$ steht,<br>worin |
| $R^{9'}$ | geradkettiges oder verzweigtes Alkyl mit bis zu 8 Kohlenstoffatomen, Benzyl oder Phenyl bedeutet, wobei letztere gegebenenfalls bis zu 3-fach gleich oder verschieden durch Halogen, Hydroxy, Nitro, Cyano, Trifluormethyl, Trifluormethoxy oder durch geradkettiges oder verzweigtes Alkyl, Alkoxy oder Alkoxycarbonyl mit jeweils bis zu 6 Kohlenstoffatomen, Carboxy oder durch die oben aufgeführte Gruppe $-NR^{7'}R^{8'}$ substitituiert sind, worin |
| $R^{7'}$ und $R^{8'}$ | die oben angegebene Bedeutung haben,<br>für Phenyl steht, das gegebenenfalls bis zu 3-fach gleich oder verschieden durch |

Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 6 Kohlenstoffatomen oder durch eine Gruppe der Formel $-NR^{7'}R^{8'}$ oder $-SO_2R^{9'}$ substituiert ist, worin

$R^{7'}$, $R^{8'}$ und $R^{9'}$ die oben angegebene Bedeutung haben,

$R^{3'}$ für Wasserstoff oder für geradkettiges oder verzweigtes Alkyl mit bis zu 8 Kohlenstoffatomen steht, das gegebenenfalls durch Phenyl substituiert ist,

oder

$R^{2'}$ und $R^{3'}$ gemeinsam für den Rest der Formel $=CHR^{10'}$ stehen,

worin

$R^{10'}$ die oben angegebene Bedeutung von $R^{5'}$ hat und mit dieser gleich oder verschieden ist,

D' für ein Sauerstoff- oder Schwefelatom oder für die >NH-Gruppe steht,

$R^{4'}$ für Wasserstoff oder für geradkettiges oder verzweigtes Alkyl mit bis zu 8 Kohlenstoffatomen oder Phenyl steht, wobei letztere gegebenenfalls bis zu 3-fach gleich oder verschieden durch Hydroxy, Halogen, Nitro, Cyano, Carboxy, Trifluormethyl, Trifluormethoxy, durch geradkettiges oder verzweigtes Alkoxy, im Fall von Phenyl auch durch Alkyl, Acyl oder Alkoxycarbonyl mit jeweils bis zu 6 Kohlenstoffatomen oder durch eine Gruppe der Formel $-NR^{7'}R^{8'}$ oder $-SO_2R^{9'}$ substituiert sind,

worin

$R^{7'}$, $R^{8'}$ und $R^{9'}$ die oben angegebene Bedeutung haben,

oder für den Fall, daß D für die >NH-Gruppe steht,

$R^{4'}$ für die Gruppe der Formel $-SO_2R^{9'}$ steht,

worin

$R^{9'}$ die oben angegebene Bedeutung hat,

mit der Maßgabe, daß wenn A' für die $-CH_2$-Gruppe, B' für die $-NR^{6'}$-Gruppe, D' für Sauerstoff und $R^{1'}$, $R^{2'}$, $R^{3'}$ und $R^{4'}$ für Wasserstoff stehen, $R^{6'}$ nicht für Wasserstoff stehen darf,

und im Fall, daß $R^{2'}$ für die tert.Butylgruppe und $R^{3'}$ für Wasserstoff steht, $R^{6'}$ nicht für die Acetylgruppe stehen darf,

und wenn B' für die $-CH_2$-Gruppe, $R^{1'}$, $R^{2'}$ und $R^{3'}$ für Wasserstoff, D' für Sauerstoff, $R^{4'}$ für Ethyl und A' für die $-NR^{6'}$-Gruppe stehen, $R^{6'}$ nicht Wasserstoff oder Benzoyl bedeuten darf.

**6.** Verfahren zur Herstellung von substituierten Pyrrolidinen der allgemeinen Formel I gemäß Anspruch 1, dadurch gekennzeichnet, daß man

[A] im Fall, daß A und A' jeweils entsprechend für die $-NR^6$- oder $-NR^{6'}$-Gruppe und B bzw. B' entsprechend für die $-CHR^5$- oder $-CHR^{5'}$ stehen,

Verbindungen der allgemeinen Formel (II)

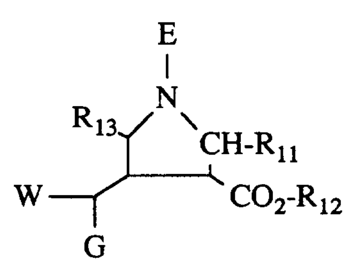 (II),

in welcher

W für eine Gruppe der Formel

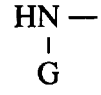

oder die $N_3$-Gruppe steht,

$R^{11}$ und $R^{13}$ entsprechend den jeweiligen Bedeutungsumfang von $R^1/R^{1'}$ und $R^5/R^{5'}$ erfassen,

E für einen $C_1$-$C_6$-Alkoxycarbonylrest steht,

G für eine Aminoschutzgruppe, vorzugsweise für Benzyl steht,
und

$R^{12}$ für $C_1$-$C_6$-Alkylrest steht,

zunächst nach üblichen Methoden durch Abspaltung der Schutzgruppe G, und im Falle, daß W für die Gruppe

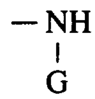

steht und im Falle der $N_3$-Gruppe durch die Reaktion mit Triophenylphosphonoxid/$H_2O$, in die Verbindungen der allgemeinen Formel (III)

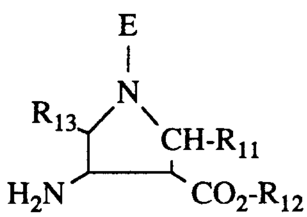 (III),

in welcher

$R^{11}$, $R^{12}$, $R^{13}$ und E die oben angegebene Bedeutung haben,

überführt,

und anschließend mit aktivierten Carbonsäurederivaten, in Anwesenheit einer Base, zu den Verbindungen der allgemeinen Formel (IV)

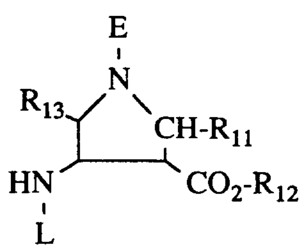 (IV),

in welcher

E, $R^{11}$, $R^{12}$ und $R^{13}$ die oben angegebene Bedeutung haben,
und

L für einen $C_1$-$C_6$-Alkoxycarbonylrest steht,

umsetzt,

und in einem letzten Schritt durch Umsetzung mit HBr in Wasser gleichzeitig unter Abspaltung der Reste E, L und $R^{12}$ sowohl die Amin- als auch die Säurefunktion freisetzt,

oder Verbindungen der allgemeinen Formel (V)

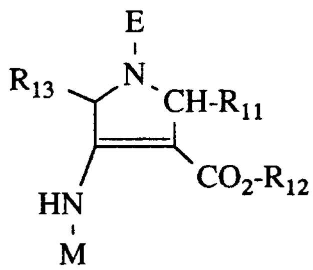

(V),

in welcher

E, $R^{11}$, $R^{12}$ und $R^{13}$ die oben angegebene Bedeutung haben,

und

M mit Ausnahme von Wasserstoff die oben angegebene Bedeutung von $R^2$ hat, vorzugsweise aber für einen $(C_1-C_6)$-Acylrest, wie Acetyl, oder für den $-CH(C_6H_4-OCH_3)_2$ steht,

in inerten Lösemitteln durch Hydrierung mit Wasserstoff in Anwesenheit eines Katalysators oder mit Hydriden zu den entsprechenden Pryrrolidinverbindungen reduziert

oder

[B] im Fall, daß A und A' entsprechend für die $-CHR^5$ oder $-CHR^{5'}$-Gruppe und B und B' entsprechend für die $-NR^6$- oder $-NR^{6'}$-Gruppe stehen,

Verbindungen der allgemeinen Formel (VI)

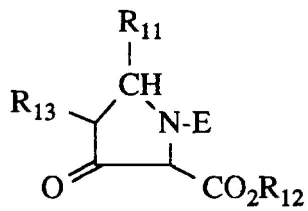

(VI),

in welcher

E, $R^{11}$, $R^{12}$ und $R^{13}$ die oben angegebene Bedeutung haben,

mit Aminen der Formel (VII)

$NH_2-R^{14}$ (VII),

in welcher

$R^{14}$ für $(C_2-C_6)$-Alkyl oder Benzyl, vorzugsweise für Benzyl steht,

in die entsprechenden Enaminoester der allgemeinen Formel (VIII)

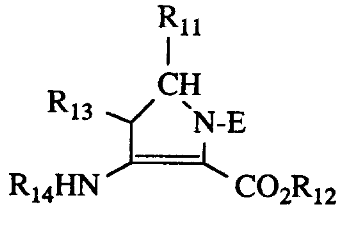

(VIII),

in welcher

E, $R^{11}$, $R^{12}$, $R^{13}$ und $R^{14}$ die oben angegebene Bedeutung haben,

überführt,

die Doppelbindung mit Wasserstoff in Anwesenheit eines Katalysators reduziert, und in einem letzten Schritt den Rest $R^{14}$ ebenfalls mit Wasserstoff in Anwesenheit eines Katalysators abspaltet,

oder
direkt Verbindungen der allgemeinen Formel (VI) durch Umsetzung mit Ammoniak oder Ammoniumsalzen und anschließend unter Einwirkung von Wasserstoff in Anwesenheit eines Katalysators, oder mit Cyanoborhydrid, in Anwesenheit von Säuren in inerten Lösernitteln reduktiv aminiert,
und im Fall, $R^2/R^3$ = H, die jeweiligen Schutzgruppen auf jeder der oben aufgeführten Stufen nach üblichen Bedingungen abspaltet,
und
im Fall der Säuren [(I), (Ia) D = O, $R^4$ = H] gegebenenfalls die entsprechenden Ester verseift,
und im Fall, der für D, D', $R^4$ und $R^{4'}$ oben aufgeführten anderen Definitionen, ebenfalls nach üblichen Verfahren, wie beispielsweise Amidierung,
Sulfonierung oder Sulfoamidierung, gegebenenfalls in Anwesenheit von Hilfsstoffen wie Katalysatoren und Dehydratisierungsmitteln, ausgehend von den entsprechenden Carbonsäuren, gegebenenfalls unter vorgeschalteter Aktivierung derivatisiert.

**7.** Arzneimittel enthaltend substituierte Pyrrolidine der allgemeinen Formel I gemäß Anspruch 1 bis 5.

**8.** Verwendung von substituierten Pyrrolidinen der allgemeinen Formel 1 gemäß der Ansprüche 1 bis 5 zur Herstellung von Arzneimitteln in der Human- oder Veterinärmedizin.

Europäisches Patentamt

# EUROPÄISCHER RECHERCHENBERICHT

Nummer der Anmeldung

EP 92 11 7263

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | WO-A-9 106 536 (THE REGENTS OF THE UNIVERSITY OF CALIFORNIA) 16. Mai 1991 | 1-8 | C07D207/16<br>A61K31/40 |
| D,X | TETRAHEDRON<br>Bd. 27, Nr. 19, 1971,<br>Seiten 4681 - 4685; 'capreomycidine and 3-guanidinoproline from viomycidine'<br>*siehe verbindungen 15,16,17* | 5 | |
| X | JOURNAL OF NEUROCHEMISTRY<br>Bd. 37, Nr. 6, 1981,<br>Seiten 1509 - 1516; 'effect of homo-beta-proline and other heterocyclic GABA analogues on GABA uptake in neurons and astroglial cells and on GABA receptor binding'<br>*siehe Table 1* | 1,2,3,5 | |
| D,X | LIEBIGS ANN. CHEM.<br>Bd. 6, 1981,<br>Seiten 1073 - 1088; 'Darstellung von cis- und trans-C-3-substituierten Prolinverbindungen'<br>*siehe Verbindungen 11a und 8I* | 5 | **RECHERCHIERTE SACHGEBIETE (Int. Cl.5 )** |
| A | JOURNAL OF ANTIBIOTICS<br>Bd. 44, Nr. 5, Mai 1991,<br>Seiten 546 - 549; 'Synthesis and antifungal activity of FR 109615 analogs'<br>----- | 1-8 | C07D<br>A61K |

Der vorliegende Recherchenbericht wurde für alle Patentansprüche erstellt

| Recherchenort | Abschlußdatum der Recherche | Prüfer |
|---|---|---|
| MUENCHEN | 11 JANUAR 1993 | SCRUTON-EVANS I. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)